# EUROPEAN PATENT SPECIFICATION

(11) **EP 1 959 876 B1**
(45) Date of publication and mention of the grant of the patent: **28.09.2011**
(21) Application number: 06818141.1
(22) Date of filing: 01.12.2006
(51) Int. Cl.: A61F 2/88, A61F 2/04

(54) **A STENT**
EIN STENT
ENDOPROTHESE VASCULAIRE

(30) Priority: 02.12.2005 DK 200501709
(43) Date of publication of application: 27.08.2008
(73) Proprietor: PNN Medical A/S, 3490 Kvistgård (DK)
(72) Inventor: SØRENSEN, Morten, DK-1420 København K (DK); HARBOE, Henrik, DK-2840 Holte (DK); OTHEL-JACOBSEN, Erik, DK-3150 Hellebæk (DK)
(74) Representative: Plougmann & Vingtoft A/S
(86) International application number: PCT/DK2006/000682
(87) International publication number: WO 2007/062661

(56) References cited:
- EP-A1- 0 631 762
- WO-A-93/13824
- WO-A-94/20044
- WO-A2-2004/032799
- US-A- 4 969 458
- US-A1- 2001 049 549
- US-A1- 2004 193 246
- US-B1- 6 416 545

## Description

### Field of invention

The present invention relates to a stent for insertion into and placement inside a body cavity of a human being or an animal, said stent being made of a material capable of being readily shaped at body cavity temperature and said stent being intended for having a first configuration during insertion into the cavity and said stent, after having been placed in the cavity, being intended for having a second configuration.

### Background

Stents are generally used for at least retaining, possibly also creating, a lumen in a body cavity. Stents are primarily shaped as a substantially tubular intraluminal prosthesis and are placed inside a body cavity of a human being or an animal. Stents may be used in a variety of body cavities, such as in the urinary canals, the blood vessels, the airways, etc., where occlusion of the body cavity may occur.

A purpose of the stent is to support the inside walls of the body cavity, to enable or preserve flow of fluids such as urine, blood or air through the body cavity if the cross-section of the body cavity is in any way narrowed, compressed, collapsed or in case the lumen of the body cavity is occluded in some other way.

Several different designs of stents have been developed, each type being especially designed for a specific use. Some existing stents have been designed as helically coiled stents, others as stents made from a web-structure, woven from wires or cut from a tube.

For the purpose of anchoring the stent in a specific position inside the body cavity, known stents may be anchored in a desired position inside the body cavity by expanding a part of the longitudinal extension of the stent or by expanding the entire longitudinal length of the stent.

US 4,969,458 describes a device to be used as a vascular stent comprising a cylindrical open-ended wire component made of a low memory metal such as copper alloy, titanium, or gold, providing a radial support from within a blood vessel after implantation therein. The coronary stent is characterized by its ability to be expanded radially to a larger diameter after initial implantation and means for causing said stent to expand to a larger diameter.

US2001/0049549 discloses a stent for use in a curved body lumen is disclosed. The stent is made from a super-elastic alloy such as nickel titanium or nitinol, and optionally includes a ternary element. The super-elastic alloy has a low temperature phase or martensitic phase and a high temperature phase or an austenitic phase. In the high temperature phase, the stent has a curve along the length that closely matches the curve of the vessel in the patient's anatomy. When deployed in the curved vessel of the patient, the heat set curve of the stent closely conforms to the curvature in the vessel and minimizes trauma and stress to the vessel. The vessel has the same diameter along the entire extension.

A disadvantage when considering many known stents is that migration of the stents inside the body of the human or animal has been observed. Such disadvantage of stents for implantation in the intraprostatic urethra being positioned between the external urethral sphincter and the bladder has been observed as the stent occasionally migrates towards a position between the colliculus and the bladder. Eventually, the stent migrates all the way to the bladder.

Another disadvantage of many known stents is that the stents have a tendency of eroding into the tissue surrounding the body cavity. Other known stents produced from metals either being super-elastic or having shape memory effect or being plastically deformable at body temperature are known to apply a pressure on the surrounding tissue of such magnitude so as to cause traumatic damages to the surrounding tissue and may also be seen eroding into the tissue surrounding the body cavity.

### Summary of the invention

An object of the present invention is to provide a stent for obtaining a satisfactory retainment of the lumen, but also obtaining a reduced risk of the stent migrating, plus the stent causing only minimal traumatic damages to the tissue surrounding the body cavity and minimal or perhaps no in-growth of the tissue surrounding the body cavity, into which the stent is to be inserted.

The object and the advantages that will be described in the following description of the invention is obtained by a stent comprising
- at least one retaining part for retaining a lumen inside the body cavity, and
- at least one expandable part for maintaining the stent in placement,
- said at least one expandable part having a first decreased cross-sectional extension in the first configuration, and said at least one expandable part having a second, increased cross-sectional extension in the second configuration, and
- said at least one retaining part in the second configuration having a cross-sectional extension, along at least part of a longitudinal extension of the retaining part, being smaller than the cross-sectional extension of at least part of the expandable part in the second configuration, and
- where the expandable part is constituted by at least one first section of at least one helical winding in a clockwise direction and at least one second section of at least one helical winding in a counter-clockwise direction, each of said first and second at least one section being radially expandable so that during expansion, the at least one first section will be rotating in counter-clockwise direction and the at least one second section will be rotating in clockwise direction, and
- where both the retaining part and the expandable part are intended for placement in a single body cavity, said single boy cavity not being provided with body organs, which is intended for at least partly blocking the body cavity, such as sphincters, membranes, orifices and similar body organs partly or wholly blocking one part of a body cavity from another part of a body cavity.

Stents are used to support the inside walls of a body cavity of a human being or animal and thereby enabling and at least retaining flow of liquid or gas through the body cavity in the occurrence of the cross-section of the cavity having in any way been narrowed, compressed, collapsed or the lumen of the cavity having in any other way been occluded.

Preferably, the retaining part in the second configuration has a cross-sectional extension, along at least part of a longitudinal extension of the retaining part, being smaller than the normal cross-sectional extension of the body cavity along said extension of the retaining part.

Preferably, at least part of the expandable part in the second configuration has a cross-sectional extension, along a longitudinal extension of the expandable part, being greater than a cross-sectional extension of the body cavity along said extension of the expandable part.

In the remainder of the present description, the part of a stent intended for retaining a lumen inside the body cavity is called a retaining part. In the case the lumen has wholly or partly collapsed, the retaining part may be placed inside the cavity by forcing open the collapsed lumen by means of the stent to enable flow, or the retaining part may be placed inside the cavity for a subsequent transformation of the shape of the stent to open the collapsed lumen.

The stent is placed inside the body cavity at a specific position defined by the position of the occluded lumen inside the human being or animal. For ensuring that the stent maintain the position during natural or inflicted movements of the human being or animal, or during natural or inflicted movements of the tissue surrounding the body cavity, the stent is pre-formed with a curvature and/or an expandable part.

By pre-formed is meant that the stent during manufacture of the stent has been provided with a certain curvature and/or has been provided with a certain expanded part. The manufacture of the stent geometry takes place when the stent is in a second configuration of the stent, said second configuration also being the configuration intended when the stent is placed inside the body cavity of the human being or animal, ready for fulfilling the purpose of retaining the lumen of the body cavity.

The curvature of the stent is intended for conforming to a curvature of the lumen of the human being or the animal. Thus, when placed in the lumen, neither the lumen nor the stent need deformation to match the profile of the lumen or of the stent, respectively. Curvature conformity results in reduced localized forces to the internal walls of the lumen.

Conforming of the curvature of the stent to the internal profile of the lumen may be accomplished by mapping the internal profile of the lumen, preferably in 3-dimensional space. Then, curvature of the stent may be formed accordingly, e.g. by heat-treating the stent or by plastically deforming the stent. Alternatively, an apparatus such as a balloon catheter may be formed and adapted for plastically deforming the stent to impose the curvature. Mapping of the body lumen may be accomplished using a variety of techniques, including ultrasound, intravascular ultrasound, angiography, radiography, magnetic resonance imaging, computed tomography and CT angiography.

As an alternative to forming the curvature of the stent or the curvature of apparatus for plastically deforming the stent, a statistical curvature matching technique may be used. The stent or the apparatus may be provided with a standardized curvature that more closely conforms to an average curvature for a specific body cavity lumen within a specific human or animal population. As with curvature conforming to the lumen, statistical curvature matching of the curvature may be facilitated or augmented by pre-mapping the intended body cavity lumen, into which the stent is intended for insertion and placement.

As a further alternative, stents may be manufactured in a number of different styles, each having its own predetermined curvature conforming to different specific body cavity lumens of a human being or animal. In this manner, a clinician may select a stent having a degree of curvature most appropriate for the specific lumen presented by the case at hand. As with curvature conforming to the lumen, predetermined curvature matching of the curvature may be facilitated or augmented by pre-mapping the different intended body cavity lumens, into which the stent is intended for insertion and placement.

Subsequent to manufacture of the stent geometry, and when the stent is to be inserted into the body cavity, the stent is transformed to a first configuration, said first configuration being the configuration intended during insertion of the stent into the body cavity of the human being or animal, before being ready for fulfilling the purpose of retaining the lumen of the body cavity.

In the embodiment of the stent, in which the retaining part is curved in the second configuration, and irrespective of the structure of the expandable part, the retaining part is preferably transformed to a substantially straight structure in the first configuration for thereby obtaining an easy insertion into the cavity in the first configuration.

According to an alternative aspect of the invention, the invention relates to a stent, in which the stent itself comprises a marker element which is detectable from outside the body cavity by at least one of the following detections: optically, tactilely, photographically, electronically or radiologically for being able of obtaining a correct physical placement of the stent in the body cavity. Preferably, the marker element of the stent itself is capable of marking a rotational orientation of the stent in respect of a longitudinal axis of the stent.

A marker element provided in conjunction with a stent delivery system or provided in conjunction with the stent itself result in the possibility of establishing the orientation of the stent after the stent has been inserted into the body cavity. Thereby, it is possible to ensure a correct placement of the stent in the body cavity, either in respect of a longitudinal orientation of the stent or in respect of a rotational orientation of the stent, or in respect of both a longitudinal orientation and rotational orientation of the stent.

A correct longitudinal orientation of the stent is especially necessary in the case where the stent is provided with at least one expandable part. It is necessary for the expandable part to be positioned correctly in relation to the body cavity so that the expandable part will expand towards the proper section of internal walls of the body cavity, which section of the internal walls must be capable of tolerating the pressure from the expandable part.

Also, a correct longitudinal orientation of the stent is necessary in the case where the stent is provided with more expandable parts. It is necessary for the expandable parts to be positioned correctly in relation to the body cavity so that the expandable parts will expand towards the proper sections of internal walls of the body cavity, which sections of the internal walls must be capable of tolerating the pressure from the expandable parts.

A correct rotational orientation of the stent is especially necessary in the case, in which the stent is provided with a curvature. It is necessary for the curvature of the stent to be positioned correctly in relation to the corresponding curvature of the body cavity lumen so that the curvature of the stent will conform to the corresponding curvature of the body cavity lumen.

Also, a correct rotational orientation of the stent is especially necessary in the case, in which the stent is provided with more curvatures. It is necessary for the different curvatures of the stent to be positioned correctly in relation to the corresponding different curvatures of the body cavity lumen so that the different curvatures of the stent will conform to the correct corresponding curvatures of the body cavity lumen.

A combined correct longitudinal orientation and correct rotational orientation is necessary where the stent is provided both with at least one expandable part and with at least one curvature.

The shape of the stent in the second configuration is not readily detectable when the stent during insertion into the body cavity is in the first configuration. Thus, in order to establish the correct placement in the body cavity, and because the shape of the stent when placed in the cavity is not possible to establish until after the stent is in placement, marker elements of the different kinds mentioned, is necessary or at least beneficial.

The type of marker element employed, i.e. whether the marker element is optically, tactilely, photographically, electronically or radiologically detectable, depends on the actual type of stent, the body cavity in which the stent is intended for placement, the detection equipment available, the detection equipment necessary or other factors, which clinical personnel will know or will want to employ during insertion of the actual stent in question. The retaining part of the stent is not intended for expanding into abutment with the inside walls of the cavity for maintaining the position of the stent after placement. The stent is maintained in position by means of the curvature of the stent conforming to the curvature of the cavity. Thereby, the curvature maintains the stent in position, and the stent is maintained in position during any natural or inflicted movements of the human being or animal itself or of the body cavity of the human being or animal.

A stent having the main features, especially the curvature, of the present invention may in principle be placed in almost any cavity of the body. However, stents having the main features of the present invention are especially suited for placement in body cavities such as the urological tract, the urethra, the biliary tract, the airways, the gastrointestinal tract or the blood vessels in the human or animal body. The stent will ensure passage of liquid, gas or solid inside the natural cavity, in which the stent is placed, and by means of the main features any migration of the stent will be eliminated or at least reduced, while at the same time eliminating or at least reducing to a minimum the traumatic damages to the tissue surrounding the body cavity and/or the in-growth to the tissue surrounding the body cavity.

In other embodiments of the present invention, the position of the stent, after having been inserted into and having been placed inside the body cavity, is maintained in position by means of the stent comprising at least one retaining part for retaining a lumen inside the body cavity, and furthermore at least one expandable part, said expandable part only constituting part of the entire longitudinal extension of the stent.

Subsequent to the stent having been inserted into and having been placed inside the body cavity, the expandable part is expanded by transformation of the structure having a second configuration exhibiting a pre-formed shape of the expandable part, said pre-formed shape of the expandable part being of a larger configuration than during insertion of the stent. The expandable part is intended for expanding outwards towards the inside walls of the body cavity so as to abut the inside walls of the body cavity.

In a preferred embodiment of the invention, the material from which the stent is made is a Shape Memory Alloy, having a transformation phase which is a transition phase of the material structure, said transition phase being present at a temperature interval above normal body cavity temperature of the animal or human being, the body cavity for which the stent is intended.

In one possible embodiment of the invention, in which the transformation to the second configuration, in which the expandable part is expanded, is performed by a thermal influence, such as by flushing the cavity with saline or similar fluid having a higher temperature than the transition temperature of the stent and thereby provoking a change of the crystalline structure of the material, resulting in a geometric change of the stent configuration.

In one preferred embodiment of the invention, the temperature interval of the transition phase of the Shape Memory Alloy is present between 37°C and 75°C, preferably between 37°C and 60°C, more preferred between 37°C and 45°C.

In an alternative embodiment of the invention, in which the stent comprises a curvature of the retaining part and an expandable part, the interval of the transition phase of the Shape Memory Alloy, which enables activation of the curvature, is present at a temperature below normal body cavity temperature, of the human being or animal, the body cavity for which the stent is intended, preferably between 0°C and 37°C, more preferably between 25°C and 37°C, most preferred between 30°C and 37°C, and the interval of the transition phase of the Shape Memory Alloy enabling activation of the expansion of the expandable part is present at a temperature above normal body cavity temperature, of the human being or animal, the body cavity for which the stent is intended, preferably between 37°C and 75°C, more preferably between 37°C and 60°C, most preferred between 37°C and 45°C.

Hereby the curvature may be obtained already before or during insertion of the stent, without applying additional heating energy from outside the cavity. The expansion of the expandable part may subsequently be activated by application of heat.

In another embodiment of the invention, the transformation to the second configuration, in which the expandable part is expanded, is performed by a mechanical influence of the stent or at least part of the stent, by releasing means compressing the stent in the first configuration, such as a cover, a casing or similar means for maintaining the stent in the first configuration. When the means for compressing the stent in the first configuration is released the stent will attain its second configuration by elastic change of the geometry, due to stress-relaxation of the stent.

In one embodiment according to the other embodiment of the invention, the material from which the stent is made is a Shape Memory Alloy, having a transformation phase which is a transition phase of the material structure, said transition phase being present at a temperature interval that makes the material super-elastic at normal body cavity temperature of the animal or human being, the body cavity for which the stent is intended.

In even another preferred embodiment of the invention, the transformation to the second configuration, in which the expandable part is expanded, is performed by a mechanical influence of the stent or at least part of the stent, by applying deformation forces onto the stent, such as inflating a balloon inside the stent. Hereby the stent is forced to attain its second configuration by plastical change of the geometry. The material from which the stent is made is in this preferred embodiment a material which is capable of retaining its plastical deformation after removal of the deformation force.

The stent having an expandable part has a first configuration before insertion into the cavity, in which first configuration the expandable part is in a compressed configuration when seen in a transverse extension of the stent in comparison to the expanded configuration of the expandable part of the stent.

The stent according to the present invention may consist of a number of windings of at least one wire. In a preferred embodiment of the stent, the windings are at least partly formed by a number of helical windings of at least one wire. In one embodiment, at least part of the retaining part is formed by a number of helical windings of at least one wire. In another embodiment, at least part of the expandable part is formed by a number of helical windings of at least one wire. In a still further embodiment, both at least part of the retaining part and at least part of the expandable part is formed by a number of helical windings.

In the embodiment of a stent, in which the retaining part is curved in the second configuration, and irrespective of the structure of the expandable part, the whole stent may be formed by a number of helical windings extending along the entire extension of the stent.

In the embodiment of a stent, in which the stent is provided with an expandable part, irrespective of whether the retaining part of the stent is straight or curved, and irrespective of the structure of the retaining part of the stent, the whole stent may also be formed by a number of helical windings extending along the entire extension of the stent,

In one embodiment of the invention, the helical windings of the expandable part has in the first configuration a pitch and after expansion of the expandable part, the helical windings of the expandable part in the second configuration has substantially the same pitch.

In another embodiment the retaining part and/or the expandable part that is formed by a number of helical windings of at least one wire and wherein the mutual distance between the windings is less than 5 mm, preferably less than 3 mm, more preferred less than 1. mm.

The retaining part may in a further embodiment of the present invention have another configuration than the rest of the stent, such as a web-structure, a woven structure made from one or more wires or filaments, or the retaining part may be constituted by a perforated tubular body. The web-structure may be constituted by a tubular body provided with cut-outs. The physical structure of the present stent can be made from wire which is wound in different patterns, such as cross-patterns, knitting-patterns or similar.

The stent of the present invention may comprise a plurality of retaining parts which are distributed along the longitudinal extension of the stent. The stent of the present invention may also comprise more than one curvature so as to conform to a cavity of the human being or animal having, as example an S-form, or even more curvatures.

The stent, when provided with one or more curvatures in the second configuration, may have one or more curvatures extending in two dimension or in three dimensions, Thus, the longitudinal axis of the stent along the one or more curvatures may extend in a single plane, i.e. in two dimensions, or the longitudinal axis of the stent along the one or more curvatures may extend in three dimensions.

Either only one curvature or only a limited number of curvatures extend in two dimensions, and the possible remainder of curvatures extend in three dimensions, or the one curvature or all curvatures extend in three dimensions. The extension of the longitudinal axis of the stent along the one or more curvatures depends on the shape of the body cavity into which the stent is to be inserted and in which the stent is to be placed.

The number of first sections and the number of second sections may vary depending on the stent design and depending on the body cavity into which the stent is to be inserted and is to be placed. Thus, the expandable part of the stent may comprise only one first section consisting of helical windings and only one second section consisting of helical windings. Alternatively, the stent may comprise only one first section consisting of one or more helical windings and a plurality of second sections, each consisting in one or more helical windings. Even in the alternative, the stent may comprise a plurality of first sections, each consisting of one or more helical windings, and only one second section consisting in one or more helical windings.

### Brief description of the drawings

In the following, the present invention will be described with reference to the accompanying drawings, in which:
Fig. 1 shows a first embodiment of a stent according to the present invention before insertion into the body cavity and having two types of helical windings, and
Fig. 2 shows the first embodiment of a stent after having been inserted into the body cavity and having, at one end, a cylindrical, expanded part of helical windings, and
Fig. 3 shows an alternative first embodiment of a stent according to the present invention before insertion into the body cavity and having two types of helical windings,
Fig. 4 shows the alternative first embodiment of a stent after having been inserted into the body cavity and having, at one end, a cylindrical, expanded part of helical windings,
Fig. 5 shows a second embodiment of a stent according to the present invention before insertion into the body cavity and having two types of helical windings, and
Fig. 6 shows the second embodiment of a stent after having been inserted into the body cavity and having, at the end, a cylindrical, expanded part of helical windings, and
Fig. 15 shows a third embodiment of a stent according to the present invention before insertion into the body cavity and having helical windings and axial windings, and
Fig. 16 shows the third embodiment of a stent after having been inserted into the body cavity and having, a conical, expanded part, and

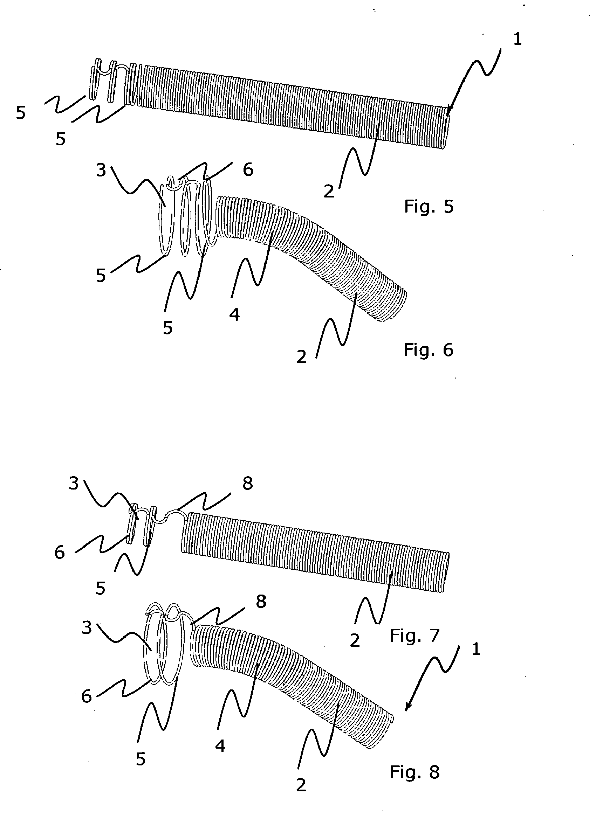

Fig. 7 shows a fourth embodiment of a stent according to the present invention before insertion into the body cavity and having two types of helical windings and a junction, and
Fig. 8 shows the fourth embodiment of a stent after having been inserted into the body cavity and having a cylindrical, expanded part of helical windings, and

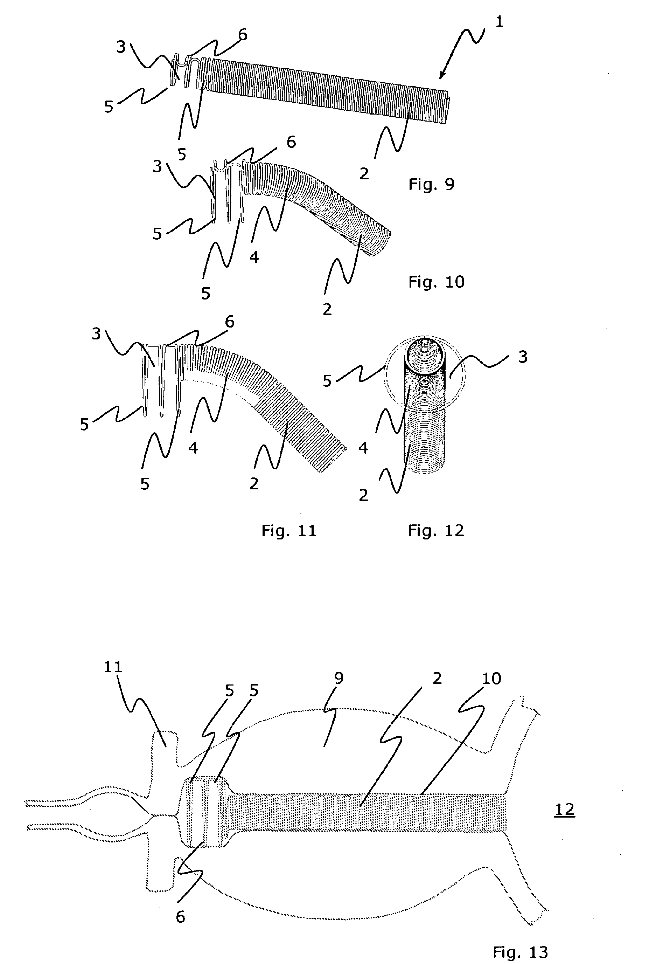

Fig. 9 shows a fifth embodiment of a stent according to the present invention before insertion into the body cavity and having two types of helical windings, and
Fig. 10 shows the fifth embodiment of a stent after having been inserted into the body cavity and having, at the end, a cylindrical, off-set expanded part of helical windings, and
Fig. 11 shows the fifth embodiment of a stent after having been inserted into the body cavity and having, at the end, the cylindrical, off-set expanded part of helical windings,
Fig. 12 also shows the fifth embodiment of a stent after having been inserted into the body cavity and having, at the end, the cylindrical, off-set expanded part of helical windings.

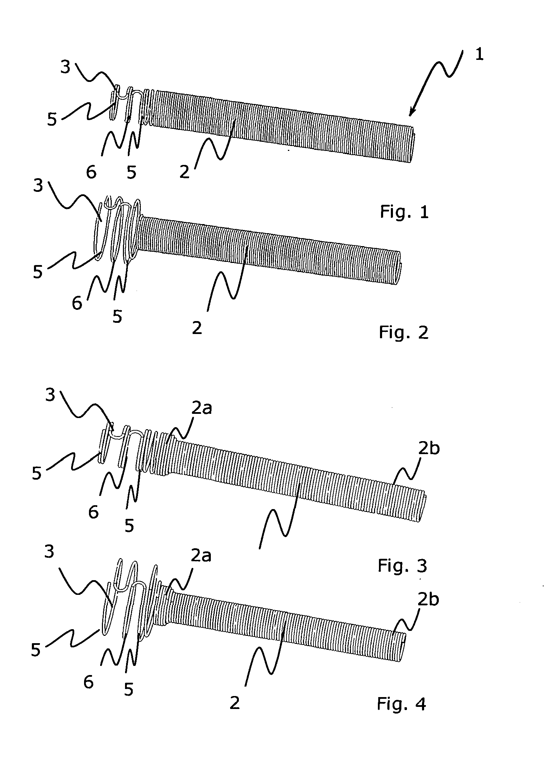

Fig. 13 shows a prostate, in which a stent according to fig. 1 and fig. 2, is positioned in the intraprostatic urethra in such a manner that both the retaining part and the expandable part is positioned in the same single body cavity.

### Detailed description of the invention

Fig. 1 and Fig. 2 show a stent 1 comprising two parts, a retaining part 2 and an expandable part 3. The stent 1 is produced by at least one metal wire which is helically wound so that the whole stent consists of a number of helical windings. In the embodiment shown, the stent is made of a Shape Memory Alloy, preferably a nickel-titanium-alloy.

The expandable part 3 and the retaining part 2 are produced so as to have the helical windings fully, or at least substantially, in abutment with each other. The retaining part 2 is designed to retain a lumen in a body cavity, as mentioned the urethra of a male human being, so as to ensure a possible flow of fluid, i.e. urine, through the lumen.

The stent 1 of Fig. 1 is shown in Fig. 2 as being straight. In the embodiment shown, the retaining part of the stent is straight both in the first configuration and in the second configuration. A transition from the retaining part 2 to the expandable part 3, and vice versa, is abrupt, i.e. no actual junction is provided between the retaining part 2 and the expandable part 3, and vice versa. Thus, there is no distance as such between the retaining part 2 and the expandable part 3, and the retaining part 2 passes directly into the expandable part 3, and vice versa.

In the embodiment shown, the retaining part 2 is shown having substantially the same cross-sectional area along the entire longitudinal extension of the retaining part.

When the stent 1, when being in the first configuration as shown in Fig. 1, is inserted into and has been positioned in the intraprostatic urethra, and the stent subsequently is transformed to the second configuration, in which expansion of the expandable part 3 of the stent 1 has occurred, the stent 1 will maintain its position without migrating, and will allow urinary passage without obstructing the valve-function of the sphincter.

Fig. 3 and Fig. 4 also show a stent 1 comprising two parts, a retaining part 2 and an expandable part 3. The stent 1 is produced by at least one metal wire which is helically wound so that the whole stent consists of a number of helical windings. In the embodiment shown, the stent is made of a Shape Memory Alloy, preferably a nickel-titanium-alloy.

The expandable part 3 and the retaining part 2 are produced so as to have the helical windings fully, or at least substantially, in abutment with each other. The retaining part 2 is designed to retain a lumen in a body cavity, as mentioned the urethra of a male human being, so as to ensure a possible flow of fluid, i.e. urine, through the lumen.

The stent 1 of Fig. 3 is shown in Fig. 4 as being straight. In the embodiment shown, the retaining part of the stent is straight both in the first configuration and in the second configuration. A transition from the retaining part 2 to the expandable part 3, and vice versa, is abrupt, i.e. no actual junction is provided between the retaining part 2 and the expandable part 3, and vice versa. Thus, there is no distance as such between the retaining part 2 and the expandable part 3, and the retaining part 2 passes directly into the expandable part 3, and vice versa.

In the embodiment shown, the retaining part 2 is shown having a proximate longitudinal extension 2a. The longitudinal extension of the retaining part extends in the immediate vicinity of the transition between the retaining part 2 and the expandable part 3. The proximate longitudinal extension 2a has a cross-sectional area being larger than the cross-sectional area of another longitudinal extension, a distant longitudinal extension 2b of the retaining part 2. The distant longitudinal extension 2b is neighbouring the proximate longitudinal extension 2a.

When the expandable part 3 expands from the first configuration to the second configuration, the expansion thus takes place from a relative larger cross-sectional area. Accordingly, the expanded cross-sectional area of the expandable part may be enlarged, due to the expansion taking place from an enlarged cross-sectional area of the retaining part. Preferably, between 1 mm and 5 mm of the retaining part 2 is constituted by the proximate longitudinal extension 2a having the relatively larger cross-sectional area, while the remaining longitudinal extension of the retaining part 2 constitutes the distant longitudinal extension 2b having the relatively smaller cross-sectional area.

When the stent 1, when being in the first configuration as shown in Fig. 3, is inserted into and has been positioned in the intraprostatic urethra, and the stent subsequently is transformed to the second configuration, in which expansion of the expandable part 3 of the stent 1 has occurred, the stent 1 will maintain its position without migrating, and will allow urinary passage without obstructing the valve-function of the sphincter.

Fig. 5 and Fig. 6 show a stent 1 comprising two parts, a retaining part 2 and an expandable part 3. The expandable part 3 is only visible in Fig. 6. In the embodiment shown, the expandable part 3 is shaped substantially as a cylinder, and the expandable part 3 is provided at an end of the stent 1. The stent 1 is produced by at least one metal wire which is helically wound so that the whole stent consists of a number of helical windings. In the embodiment shown, the stent is made of a Shape Memory Alloy, preferably a nickel-titanium-alloy.

The retaining part 2 is produced so as to have the helical windings fully, or at least substantially, in abutment with each other. The expandable part 3 is produced so as to have the helical windings spaced apart from each other. A transition from the retaining part 2 to the expandable part 3, and vice versa, is abrupt, i.e. no actual junction is provided between the retaining part 2 and the expandable part 3, and vice versa. Thus, there is no distance as such between the retaining part 2 and the expandable part 3, and the retaining part 2 passes directly into the expandable part 3, and vice versa.

The retaining part 2 is designed to retain a lumen in a body cavity, as mentioned the urethra of a male human being, so as to ensure a possible flow of fluid, i.e. urine, through the lumen. The retaining part 2 is furthermore designed with a curvature 4 in the second configuration to conform to a corresponding curvature of the body cavity, i.e. the intraprostatic curve in the urethra.

The stent 1 of Fig. 5 is shown in Fig. 6 having a curvature 4. In the embodiment shown, the retaining part 2 of the stent 1 is straight in the first configuration, and the curvature 4 is only provided in the second configuration. In an alternative embodiment, the retaining part 2 of the stent 1 is already provided with the curvature 4 in the first configuration.

In the embodiment shown, the retaining part 2 is shown having substantially the same cross-sectional area along the entire longitudinal extension of the retaining part. In an alternative embodiment, a proximate longitudinal extension is provided similar to the proximate longitudinal extension 2a shown in fig. 3 and fig. 6. Accordingly, in the alternative embodiment of fig. 5 and fig. 6, the longitudinal extension of the retaining part extends in the immediate vicinity of the transition between the retaining part and the expandable part. The proximate longitudinal extension has a cross-sectional area being larger than the cross-sectional area of another longitudinal extension, a distant longitudinal extension of the retaining part. The distant longitudinal extension is neighbouring the proximate longitudinal extension.

In the alternative embodiment of fig. 5 and fig. 6, when the expandable part expands from the first configuration to the second configuration, the expansion thus takes place from a relative larger cross-sectional area. Accordingly, the expanded cross-sectional area of the expandable part may be enlarged, due to the expansion taking place from an enlarged cross-sectional area of the retaining part. Preferably, between 1 mm and 5 mm of the retaining part the proximate longitudinal extension having the relatively larger cross-sectional area, while the remaining longitudinal extension of the retaining part constitutes the distant longitudinal extension having the relatively smaller cross-sectional area.

When the stent 1, when being in the first configuration as shown in Fig. 5, is inserted into and has been positioned in the intraprostatic urethra, and the stent subsequently is transformed to the second configuration, in which expansion of the expandable part 3 of the stent 1 has occurred, the stent 1 will maintain its position without migrating, and will allow urinary passage without obstructing the valve-function of the sphincter.

Fig. 7 and Fig. 8 show a stent 1 comprising two parts, a retaining part 2 and an expandable part 3. The expandable part 3 is only visible in the expanded configuration in Fig. 8. In the embodiment shown, the expandable part 3 is shaped substantially as a cylinder, and the expandable part 3 is provided at an end of the stent 1.

A junction 8 is provided between the retaining part 2 and the expandable part 3. The junction 8 is maintaining a mutual rotational relationship between the retaining part and the expandable part, both in the first configuration of Fig. 7 and in the second configuration of Fig. 8. A transition from the retaining part 2 to the expandable part 3, and vice versa, extends over a certain distance. Thus, a distance, provided by the junction, is present between the retaining part 2 and the expandable part 3, and the retaining part 2 passes into the expandable part 3, and vice versa, along the junction. A stent, which is provided with a curvature, may have the curvature of the retaining part being positioned in immediate vicinity of a junction between the retaining part 2 and the expandable part 3.

The distance provided by the junction between the retaining part and the expandable part has a value between 0 mm and 50 mm, and preferably between 0 mm and 20 mm, possibly between 0 mm and 10 mm, even possibly between 0 mm and 5 mm, at least in the second configuration shown in fig. 8. The distance of 0 mm is the case, where the junction extends in a plane perpendicular to a longitudinal axis along the transition between the retaining part 2 and the expandable part 3. The distance between the retaining part 2 and the expandable part 3 is preferably selected so that the retaining part 2 and the expandable part 3, when placed in the body cavity, is capable of being placed in a single body cavity not being provided with body organs, which is intended for at least partly blocking the body cavity, such as sphincters, membranes, orifices and similar body organs partly or wholly blocking one part of a body cavity from another part of a body cavity. Such different body organs intended for at least partly blocking the body cavity in effect divide the body cavity into two minor body cavities, i.e. different from a major single body cavity.

The stent 1 is produced by at least one metal wire which is helically wound so that most of the stent consists of a number of helical windings. In the embodiment shown, the stent is made of a Shape Memory Alloy, preferably a nickel-titanium-alloy.

The retaining part 2 is produced so as to have the helical windings fully, or at least substantially, in abutment with each other. The expandable part 3 is produced so as to have the helical windings spaced apart from each other. The retaining part 2 is designed to retain a lumen in a body cavity, as mentioned the urethra of a male human being, so as to ensure a possible flow of fluid, i.e. urine, through the lumen. The retaining part 2 is furthermore designed with a curvature 4 in the second configuration to conform to a corresponding curvature of the body cavity, i.e. the intraprostatic curve in the urethra.

The stent 1 of Fig. 7 is shown in Fig. 8 having a curvature 4. In the embodiment shown, the retaining part 2 of the stent 1 is straight in the first configuration, and the curvature 4 is only provided in the second configuration. In an alternative embodiment, the retaining part 2 of the stent 1 is already provided with the curvature 4 in the first configuration.

When the stent 1, when being in the first configuration as shown in Fig. 7, is inserted into and has been positioned in the intraprostatic urethra, and the stent subsequently is transformed to the second configuration, in which expansion of the expandable part 3 of the stent 1 has occurred, the stent 1 will maintain its position without migrating, and will allow urinary passage without obstructing the valve-function of the sphincter.

Fig. 9, Fig. 10, Fig. 11 and Fig. 12 show a stent 1 comprising two parts, a retaining part 2 and an expandable part 3. In the embodiment shown, the expandable part 3 is shaped substantially as a cylinder, and the expandable part 3 is provided at an end of the stent 1. The expandable part is, in the second configuration, provided off-set in relation to the retaining part 2. The off-set provision of the expandable part 3 result in a central longitudinal axis (not shown) of the retaining part 2, when the expandable part is expanded as shown in Fig. 10, Fig. 11 and Fig. 12, is not intersecting, in the passing from the retaining part to the expandable part, a central longitudinal axis (not shown) of the expandable part. The stent 1 is produced by at least one metal wire which is helically wound so that most of the stent consists of a number of helical windings. In the embodiment shown, the stent is made of a Shape Memory Alloy, preferably a nickel-titanium-alloy.

The retaining part 2 is produced so as to have the helical windings fully, or at least substantially, in abutment with each other. The expandable part 3 is produced so as to have the helical windings spaced apart from each other. The retaining part 2 is designed to retain a lumen in a body cavity, as mentioned the urethra of a male human being, so as to ensure a possible flow of fluid, i.e. urine, through the lumen. The retaining part 2 is furthermore designed with a curvature 4 in the second configuration to conform to a corresponding curvature of the body cavity, i.e. the intraprostatic curve in the urethra.

The stent 1 of Fig. 9 is shown in Fig. 10, Fig. 11 and Fig. 12 having a curvature 4. In the embodiment shown, the retaining part 2 of the stent 1 is straight in the first configuration, and the curvature 4 is only provided in the second configuration. In an alternative embodiment, the retaining part 2 of the stent 1 is already provided with the curvature 4 in the first configuration.

As mentioned, the central longitudinal axis of the expanding part 3 is offset from the central axis of the retaining part. The embodiment is characterised by not having any bends in the longitudinal direction. This results in an ability to attain the same degree and direction of strain in all elements of the expanding sections during the change of geometry.

Furthermore, the embodiment is characterised by having a geometry that will force the retaining part 2 of the stent to be forced towards the bending side of the body cavity, in which the stent has been inserted, thereby resulting in an increased guarantee of the retaining part 2 not moving in a transversal extension of the stent, i.e. a direction of the stent lying in the plane of Fig. 12, showing a cross-sectional view.

In the embodiments shown in fig. 9, fig. 10, fig. 11 and fig. 12, the retaining part 2 is shown having substantially the same cross-sectional area along the entire longitudinal extension of the retaining part. In alternative embodiments, a proximate longitudinal extension is provided similar to the proximate longitudinal extension 2a shown in fig. 3 and fig. 4. Accordingly, in the alternative embodiment of fig. 9, fig. 10, fig. 11 and fig. 12, the longitudinal extension of the retaining part extends in the immediate vicinity of the transition between the retaining part and the expandable part. The proximate longitudinal extension has a cross-sectional area being larger than the cross-sectional area of another longitudinal extension, a distant longitudinal extension of the retaining part. The distant longitudinal extension is neighbouring the proximate longitudinal extension.

In the alternative embodiment of fig. 9, fig. 10, fig. 11 and fig. 12, when the expandable part expands from the first configuration to the second configuration, the expansion thus takes place from a relative larger cross-sectional area. Accordingly, the expanded cross-sectional area of the expandable part may be enlarged, due to the expansion taking place from an enlarged cross-sectional area of the retaining part. Preferably, between 1 mm and 5 mm of the retaining part the proximate longitudinal extension having the relatively larger cross-sectional area, while the remaining longitudinal extension of the retaining part constitutes the distant longitudinal extension having the relatively smaller cross-sectional area.

When the stent 1, when being in the first configuration as shown in Fig. 9, is inserted into and has been positioned in the intraprostatic urethra, and the stent subsequently is transformed to the second configuration, in which expansion of the expandable part 3 of the stent 1 has occurred, the stent 1 will maintain its position without migrating, and will allow urinary passage without obstructing the valve-function of the sphincter.

At least the retaining part of the stent 1 has a plurality of neighbouring helical windings which are substantially or fully in abutment with each other, at least in the first configuration. In the second configuration, at least part of the helical windings are still substantially or fully in abutment with each other after the material of the stent has been transformed to the second configuration, preferably by passing the transition phase of a Shape Memory Alloy.

In respect of the embodiments of the stent having one or more expandable parts 3, the one or more expandable parts 3 of the stent 1, in the second configuration, has either expanded conically and helically outwards (see Fig. 7 and 8, Fig. 9 and 10, Fig. 11 and 12), or has expanded cylindrically and helically outwards (see Fig. 1 and 2, Fig. 3 and 4, Fig. 5 and 6, Fig. 7 and 8, Fig. 9-12), or has expanded conically and radially outwards (see Fig. 15 and 16), from a longitudinal axis of the stent 1, and when the stent is in placement inside the body cavity, the expandable part 3 will abut inside walls of the body cavity of the human being or animal for anchoring the stent 1 in a specific position inside the body cavity.

The one or more expandable parts 3 of the stent 1 is shown having a first decreased cross-sectional extension in the first configuration, and the one or more expandable parts 3 is shown having a second, increased cross-sectional extension in the second configuration, so that said one or more expandable parts 3 during insertion, when having the first decreased cross-sectional configuration, is capable of passing an orifice of the body cavity, and so that said one or more expandable parts 3 after placement is capable of abutting inside walls of the body cavity.

The retaining part 2 of the stent 1 has a cross-section and possibly a curvature 4 that correspond to a normal cross-section and a possible normal curvature of the corresponding body cavity, in which the stent is to be placed. The expandable part 3 exhibits the expanded, second configuration for anchoring the stent 1 in a specific position inside the body cavity so that the stent 1 maintain its position, also during any movement of the human being or animal, in which the stent has been inserted into and is placed inside.

The one or more expandable parts 3 may have many different shapes in the expanded, second configuration. Also, all embodiments shown may, as an alternative to the actual shape shown, have one or more expandable parts 3. Furthermore, the one or more expandable parts 3 may be positioned at any desired location along the longitudinal extension of the stent 1. The one or more expandable parts 3 may be provided at one or at both ends of the stent. The one or more expandable parts 3 may be positioned at the middle of the stent 1, or the one or more expandable parts 3 may be positioned at any other location between the ends of the stent 1. Also, more than one expandable part 3 may be positioned between the ends of the stent. The number of expandable parts 3 and the location of the one or more expandable parts 3 depend on the actual application of the stent, i.e. the shape and the physical function of the body cavity, into which the stent is to be inserted and in which the stent is intended for placement.

Transition of the Shape Memory Alloy occurs by the material turning from a martensite phase to an austenite phase by the influence of applying heat or by releasing a mechanical retainment, thereby obtaining a stress-relaxation at least of the part of the stent intended for having another configuration in the second configuration of the stent. Part of the transition from martensite phase to austenite phase may occur already during insertion because of the stent 1 being heated by the temperature from the body of the human being or animal.

The transition phase of the stent 1 between the first configuration and the second configuration of the stent may, as mentioned, be passed by thermally or mechanically influencing at least a part of the stent 1. In an embodiment of the present invention, at least part of the stent 1, such as the expandable part 3 or the retaining part 2, or such as part of the expandable part 3 or part of the retaining part 2, may be heated by the use of electricity, induction heating, conduction heating, immersion heating, application of RF energy or by being flushed by a warm fluid such as sterile water, saline water or other liquids or such as any suitable gas, thereby obtaining thermal change of the crystalline structure of the material of the stent.

In another embodiment of the present invention, at least part of the stent 1, such as the expandable part 3 or the retaining part 2, or such as part of the expandable part 3 or part of the retaining part 2, may be retained in its first configuration, facilitated by the super-elastic properties of the nickel-titanium, using a cover, a casing or similar means for retaining the stent in the first configuration. When the means for retaining the stent in the first configuration is released, the stent will attain its second configuration by elastic change of the geometry, due to stress-relaxation of the stent.

In another embodiment, the stent 1 is transformed from the first configuration to the second configuration by inflating an inflatable means, such as a balloon within the stent 1. The transformation phase may also be passed by application of other kinds of mechanical manipulation, such as stretchirfg or bending, of the expandable part 3 or the retaining part 2 of the stent 1, resulting in plastical deformation of the material of the stent.

The different designs of the expandable part 3 allow for different expansion patterns. The expandable part 3 of the stent 1 of Fig. 7 and 8, of Fig. 9 and 10, and of Fig. 11 and 12 expands in a combined radial and conical pattern. The expandable part 3 of the stent 1 of Fig. 1 and 2, of Fig. 3 and 4, of Fig. 5 and 6, of Fig. 7 and 8, and of Fig. 9-12 expands in a primarily radial pattern. The expandable part 3 of the stent 1 of Fig. 7 and 8 expands in a combined radial and conical pattern. However, mutual to the expansion pattern of the expandable part 3 of the stent of Fig. 7 and 8, of Fig. 9 and 10, and of Fig. 11 and 12 and of Fig. 1 and 2, of Fig. 5 and 6, of Fig. 7 and 8, and of Fig. 9-12, the expansion pattern is primarily radial compared to any axial or other direction of expansion.

The expandable part 3 of the stent 1 in Fig. 7 and 8, of Fig. 9 and 10, and of Fig. 11 and 12 also expands tangentially around the longitudinal axis of the stent 1 into a substantially conical configuration of the expandable part. The expandable part 3 of the stent 1 in Fig. 1 and 2, of Fig. 3 and 4, of Fig. 5 and 6, of Fig. 7 and 8, and of Fig. 9-12 also expands tangentially around the longitudinal axis of the stent 1 into a substantial cylindrical configuration of the expandable part. In an alternative embodiment, the expandable part 3 of the stent 1 in Fig. 1 and 2, of Fig. 3 and 4, of Fig. 5 and 6, of Fig. 7 and 8, and of Fig. 9-12 may also expand primarily radially and also tangentially around the longitudinal axis of the stent 1 into a conical configuration of the expandable part.

The primarily radial expansion of the expandable part shown in Fig. 1 and 2, of Fig. 3 and 4, of Fig. 5 and 6, of Fig. 7 and 8, and of Fig. 9-12 is due to the fact that the expandable part 3 is constituted by at least a first section 5 of at least one helical winding which is wound in a clockwise direction and at least a second section 6 of at least one helical windings which is wound in a counter-clockwise direction; Each of said sections 5,6 are being radially expandable so that during expansion, the first section 5 will be rotating in a counter-clockwise direction and the second section 6 will be rotating in a clockwise direction.

The stent 1 of Fig. 7 and 8 is shown having a junction between the expandable part 3 and the retaining part 2. The junction will, in the second configuration, maintain a mutual positional relationship between the retaining part 2 and the expandable part 3, said positional relationship being substantially the same mutual positional relationship between the retaining part and the expandable part as in the first configuration.

The junction results in that, subsequent to having been inserted into and subsequent to having been placed in the body cavity, and during expansion of the expandable part from the first configuration to the second configuration, the expandable part and the retaining part maintain a mutual positional relationship along the longitudinal axis of the stent.

In the embodiment shown, the mutual positional relationship being maintained by the junction is both a mutual longitudinal relationship and a mutual rotational relationship. In an alternative embodiment, the junction is only intended for and is therefore only capable of maintaining either a mutual longitudinal relationship or a mutual positional relationship between the retaining part and the expandable part.

The embodiments shown in figure 1-16, and fig. 9-12, i.e. the embodiments not explicitly showing a junction, may, in alternative embodiments based on the explicitly shown embodiments, be provided with a junction between the retaining part and the expandable part. Thus, a distance, provided by a junction, may be present between the retaining part and the expandable part, also for the embodiments shown in fig. 1-16 and fig. 9-12, and the retaining part may thus pass into the expandable part, and vice versa, along a junction.

The distance provided by a possible junction between the retaining part and the expandable part has a value between 0 mm and 50 mm, and preferably between 0 mm and 20 mm, possibly between 0 mm and 10 mm, even possibly between 0 mm and 5 mm, at least in the second configuration. The distance of 0 mm is the case, in which the junction 8 extends in a plane perpendicular to a longitudinal axis along the transition between the retaining part 2 and the expandable part 3. The distance between the retaining part 2 and the expandable part 3 is preferably selected so that the retaining part 2 and the expandable part 3, when placed in the body cavity, is capable of being placed in a single body cavity not being provided with body organs, which is intended for at least partly blocking the body cavity, such as sphincters, membranes, orifices and similar body organs partly or wholly blocking one part of a body cavity from another part of a body cavity. Such different body organs intended for at least partly blocking the body cavity in effect divide the body cavity into two minor body cavities, i.e. different from a major single body cavity.

Fig. 13 shows a prostate 9, where a stent according to fig. 1 and fig. 2, is positioned in the intraprostatic urethra 10 in such a manner that both the retaining part 2 and the expandable part 3 is positioned in the same single body cavity which the intraprostatic urethra 10 constitute. Thus, both the retaining part 2 and the expandable part 3 of the stent is placed on the one side of the external urethral sphincter 11, and placed between the external urethral sphincter 11 and the bladder 12, the where said external urethral sphincter constitutes at least a partial blocking of the overall body cavity of the urinal duct.

In possible embodiments according to any of the stent designs shown in the figures and as described, the expandable part 3 of the stent 1 may be placed on one side of perhaps a muscle, such as the external urethral sphincter or other organ preferred not to be exposed to traumatic damages, and the retaining part 2 may be placed on the other side of said muscle without the muscle or other organ being applied any torsion during the expansion of the expandable part. Any possible inconvenience to the human being or animal, when the expandable part is expanded and subsequent to expansion of the expandable part, is thereby decreased or even eliminated.

Any curvature 4 of the stent according to the invention may be provided in the junction between the expandable part 3 and the retaining part 2, if any corresponding curvature of body cavity will be present in a position of the junction, when the stent is in placement in the body cavity.

The stent 1 is in most embodiments shown as having only one expandable part situated in one end of the stent and only one retaining part constituting the remainder of the stent. The stent 1 may in other embodiments according to the present invention comprise a plurality of lumen retaining parts 2 and/or a plurality of expandable parts 3 distributed along the longitudinal length of the stent 1.

In one embodiment, the stent 1 may have an expandable part 3 situated in both ends of the stent and may have one retaining part 2 of the stent 1 situated between the expandable parts 3. In another embodiment, the stent 1 may have a retaining part 2 situated in both ends of the stent and may have one expandable part 3 of the stent 1 situated between the retaining parts.

The stents of the present invention is preferably made from a Shape Memory Alloy such as Nickel-Titanium-alloy (Ni-Ti Alloy), but may also be made from other Shape Memory Alloys such as Gold-Cadmium-alloy (Au-Cd Alloy), Copper-Zink-alloy (Cu-Zn Alloy), Indium-Titanium-alloy (In-Ti Alloy), Copper-Zink-Silver-alloy (Cu-Zn-Al Alloy) or other metal alloys exhibiting shape memory characteristics.

Preferably, the Shape Memory Alloy chosen has a shape memory effect above normal body cavity temperature so that the shape of the second configuration has to be inflicted thermally for obtaining the second configuration of the stent. Alternatively, the Shape Memory Alloy chosen may have super-elastic effect at normal body cavity temperature.

In other embodiments of the present invention, the stent may be made from a material being plastically deployable at body cavity temperature such as stainless steel. In even other embodiments of the present invention, the stent may be made of a combination of different materials in order to suit a specific application of the stent. Thus, a combination of different Shape Memory Alloys may be envisaged, or a combination of one or more Shape Memory Alloys and one or more plastically deployable materials may be envisaged. In one embodiment the Shape Memory Alloy is an alloy with a transition temperature at normal body cavity temperature of the human being or animal, the body cavity for which the stent 1 is intended. Such an embodiment may be applied where the body cavity is relatively large or is very flexible so that a transformation is possible from the first configuration of the stent to the second configuration of the stent already during insertion. The term Shape Memory Alloy is defined as a metal having transformation from one crystalline phase to another crystalline phase, induced by heating or mechanical stress to the material.

In an alternative embodiment of the stent, containing both a curvature on the retaining part and an expandable part, the Shape Memory Alloy has different transition temperatures defining the activation of the curvature and expansion respectively. In this embodiment of the invention the interval of the transition phase of the Shape Memory Alloy enabling activation of the curvature is present at a temperature below normal body cavity temperature, of the human being or animal, the body cavity for which the stent is intended, and the interval of the transition phase of the Shape Memory Alloy enabling activation of the expansion of the expandable part is present at a temperature above normal body cavity temperature, of the human being or animal, the body cavity for which the stent is intended.

Hereby the curvature may be obtained already before or during insertion of a stent, without applying additional heating energy from outside the cavity. The expansion of the expandable part may subsequently be activated by application of heat.

If the Shape Memory Alloy is a Ni-Ti Alloy the crystalline phase in the first configuration of the material is martensite, and the crystalline phase in the second configuration of the material is austenite. The transformation occurs at a certain temperature range (Austenite Start to Austenite Finish (AS to AF)). Within this temperature range (AS to AF) the expansion of at least part of the stent 1 is initiated and the expansion terminates, when the martensite is transformed into austenite. The stent 1 "remembers" at this temperature range (AS to AF) its original shape, i.e. the pre-formed design that the stent 1 was given during manufacture, before the stent 1 was transformed to the first configuration for enabling insertion into a body cavity.

At another temperature range (Martensite Start to Martensite Finish (MS to MF)) the alloy reverts to the martensite phase. Below this other temperature (MF), the stent 1 is plastically deformable by hand, and the stent 1 may therefore be deformed inside the body cavity. The shape of the deformed stent inside the body cavity may be maintained after deformation, and if the deformed stent has a shape such as a helix, but being elongated, or even having the shape of a elongate wire, and thus having, in the deformed configuration, a reduced cross-sectional area compared to the cross-sectional area in the second configuration, the stent may be retracted through any natural body orifice into which the stent 1 was inserted. Alternatively the stent 1 may be retracted through another natural body orifice than the one through which it was inserted.

The term Shape Memory Alloy may also be a Ni-Ti alloy having super-elastic properties at a certain temperature, such as about 37°C and plasticity at another temperature, such as below 0°C. By the wording super-elastic properties is meant an alloy which is elastically deformable until a high level of strain (up to approximately 5%-10%)

The human or animal normal body cavity temperature may vary from human being to human being and from animal to animal. Also, the body cavity temperature may vary depending on which organ the stent is to be inserted into, However, one human being or animal normally has one average body temperature such as around 37 °C for a human being. In another aspect of the present invention the stent 1 may be made of a Shape Memory Alloy having a transition temperature between 37°C and 50°C. It is important that the transition temperature in most applications is substantially above the body temperature in that it is not convenient that the stent 1 transforms from the first configuration to the second configuration before the stent 1 is in place inside the body cavity.

The stents of the present invention are primarily produced by using at least one of the different production principles following below, resulting in different possibilities for obtaining the aforementioned curvature 4 and/or for obtaining the expansion of at least part of the stent 1, when it is placed in the desired position inside the body cavity:
1) The stent 1 may in one embodiment be produced in Shape Memory Alloy such as Ni-Ti with a transition temperature above body cavity temperature, in which the transition temperature is the temperature at which the material, of the stent 1, changes from martensite phase to austenite phase. The stent 1 is shaped into a non-curvature and/or a low-cross-sectional configuration, in a low temperature state having a martensite structure. After insertion of the stent 1 to its desired position, heat is applied to the stent, thereby elevating the temperature of the stent 1 to above the transition temperature. This results in a transformation of the material from martensite phase to austenite phase causing the stent 1 to change to the second configuration having a curvature 4 of the retaining part and/or having an expanded expandable part 3.
2) The stent 1 may in another embodiment be produced in Shape Memory Alloy such as Ni-Ti, which is super-elastic at body cavity temperature. The stent 1 is shaped into a non-curvature and/or a low-cross-sectional configuration and this low-cross-sectional configuration is mechanically retained during the insertion of the stent 1. After insertion of the stent 1 to its desired position, the mechanical retaining of the stent 1 in the low-cross-sectional configuration is released causing the stent 1 to change to the second configuration having a curvature 4 of the retaining part and/or having an expanded expandable part 3.
3) Additionally, the stent 1 may also be produced in a material that is plastically deformable, such as stainless steel. The stent 1 is shaped into a non-curvature and/or a low-cross-sectional configuration. After inserting the stent 1 to its desired position the stent 1 is plastically deformed to a high-diameter configuration by applying a pressure on the inside wall of the stent 1. This may be done by inflating a balloon inside the stent 1.

The three different principles of transformation form the first configuration to the second configuration is in the figures illustrated based on a stent 1 having a circular cross-section. In other embodiments of the present invention, the stent 1 may have another cross-sectional configuration such as oval or polygonal. However, the different principles of transformation from the first configuration to the second configuration may still be employed to such non-circular cross-sections.

According to an embodiment of the present invention the stent 1 consists of preferably a number of helical windings of only one wire. A stent constituted by only one wire is of particular advantage, when the stent 1 is to be removed or retracted from the body cavity. If the stent is made of a Shape Memory Alloy, and when the temperature of the wire-material of a stent is reduced to below the transition temperature, the stent 1 may be easily removed from the body cavity by grasping any part of the wire and subsequently pulling the wire out of the cavity as one substantial straight wire. This is especially applicable when the stent 1 is produced in Shape Memory Alloy with a transition temperature above the body cavity temperature of the human being or animal in which the stent 1 has been inserted.

The present invention further relates to a stent system comprising a stent 1 according to any of the embodiments shown and described, and further comprising a delivery device for insertion of the stent 1 into a body cavity of a human being or an animal.

Additionally, the present invention when being provided as part of a delivery device of the stent system according to the invention, said delivery system may comprise a marker element being optically, tactilely, photographically, electronically or radiologically visible from outside the body cavity for being capable of obtaining a correct physical placement of the stent 1.

Alternatively, the present invention may relate to a stent as such according to any of the embodiments shown and described, and further comprising a marker element, on the stent itself, being optically, tactilely, photographically, electronically or radiologically visible from outside the body cavity for being capable of obtaining a correct physical placement of the stent 1.

A correct physical placement of the stent 1 may be a correct longitudinal placement of the stent in the body cavity or a correct rotational placement of the stent in the body cavity or both a correct longitudinal placement and a correct rotational placement of the stent in the body cavity. A correct longitudinal placement of the stent is important when the stent has an expandable part intended for abutment with a specific part of internal walls of the body cavity, A correct rotational placement of the stent is important when the stent is provided with a curvature intended for conforming to a corresponding curvature of the body cavity.

## Claims

1. A stent (1) for insertion into and placement inside a body cavity of a human being or an animal, said stent (1) being intended for having a first configuration during insertion, and said stent(l), after having been inserted into and when being placed in the body cavity, being intended for having a second configuration, wherein said stent (1) comprises
- at least one retaining part (2,2a,2b) for retaining a lumen inside the body cavity, and
- at least one expandable part (3) for maintaining the stent (1) in placement,
- said at least one expandable part having a first decreased cross-sectional extension in the first configuration, and said at least one expandable part having a second, increased cross-sectional extension in the second configuration, and
- said at least one retaining part in the second configuration having a cross-sectional extension, along at least part of a longitudinal extension of the retaining part, being smaller than the cross-sectional extension of at least part of the expandable part in the second configuration, and
- where the expandable part (3) is constituted by at least one first section of at least one helical winding in a clockwise direction and at least one second section of at least one helical winding in a counter-clockwise direction, each of said first and second at least one section being radially expandable so that during expansion, the at least one first section will be rotating in counter-clockwise direction and the at least one second section will be rotating in clockwise direction, and
- where both the retaining part (2,2a,2b) and the expandable part (3) are intended for placement in a single body cavity, said single body cavity not being provided with body organs, which is intended for at least partly blocking the body cavity, such as sphincters, membranes, orifices and similar body organs partly or wholly blocking one part of a body cavity from another part of a body cavity.

2. A stent (1) according to claim 1, wherein at least part of the whole stent (1) is formed by a number of helical windings of at least one wire.

3. A stent (1) according to claim 1, wherein at least part of the retaining part (2,2a,2b) is formed by a number of helical windings of at least one wire.

4. A stent (1) according to claim 1, wherein at least part of the expandable part (3) is formed by a number of helical windings of at least one wire.

5. A stent (1) according to any of the preceding claims,
- where the retaining part (2,2a,2b) at least has a proximate longitudinal extension and at least has a neighbouring, distant longitudinal extension, and
- where the proximate longitudinal extension of the retaining part (2,2a,2b) extends proximate to the expandable part (3), and
- where the neighbouring distant longitudinal extension of the retaining part (2,2a,2b) extends in immediate vicinity of the proximate longitudinal extension, and
- where a cross-sectional area of the proximate longitudinal extension is greater than a cross-sectional area of the neighbouring, distant longitudinal extension.

6. A stent (1) according to any of the preceding claims, wherein the stent (1) has a junction (8) between the expandable part (3) and the retaining part (2,2a,2b), and wherein said junction (8) in the first configuration of the stent (1) is capable of maintaining a mutual positional relationship between the retaining part (2,2a,2b) and the expandable part (3), and wherein said junction (8) in the second configuration is capable of maintaining substantially the same mutual positional relationship between the retaining part (2,2a,2b) and the expandable part (3).

7. A stent (1) according to claim 6, where the junction (8) in the first configuration is capable of maintaining a mutual rotational relationship between the retaining part (2,2a,2b) and the expandable part (3), and wherein said junction (8), in the second configuration, is capable of maintaining substantially the same mutual rotational relationship between the retaining part (2,2a,2b) and the expandable part (3).

8. A stent (1) according to any of the preceding claims, wherein windings of the expandable part (3) in the first configuration has a longitudinal extension, and where the expandable part (3) in the second configuration has substantially the same longitudinal extension as in the first configuration.

9. A stent (1) according to any of the preceding claims, wherein at least part of the retaining part is formed by a number of helical windings of at least one wire and wherein the mutual distance between the windings is less than 5 mm, preferably less than 3 mm, more preferred 1 mm.

10. A stent (1) according to any of the preceding claims, wherein the material from which the stent (1) is made in the first configuration has a first phase and in the second configuration has a second phase, and where a transition phase of the material is present between the first configuration and the second configuration, and where introduction of the transition between the first configuration and the second configuration may be effected by heating the stent (1).

11. A stent (1) according to claim 1-9, wherein the material from which the stent (1) is made in the first configuration has a first phase and in the second configuration has a second phase, and where a transition phase of the material is present between the first configuration and the second configuration, and where introduction of the transition between the first configuration and the second configuration may be effected by mechanical release of the stent (1).

12. A stent (1) according to any of the preceding claims, wherein the retaining part (2,2a,2b) has a first cross-sectional extension, when configured in the first configuration, and where said first cross-sectional extension is substantially the same as a second cross-sectional extension of the retaining part (2,2a,2b), when being configured in the second configuration.

13. A stent (1) according to any of the preceding claims, wherein a central axis of the expandable part (3) and a central axis of the retaining part (2,2a,2b), when the stent (1) is in the second configuration, are intersecting at a passing between the expandable part (3) and the retaining part (2,2a,2b).

14. A stent (1) according to any of the preceding claims, wherein a central axis of the expandable part (3) and a central axis of the retaining part (2,2a,2b), when the stent (1) is in the second configuration, are off-set and not intersecting at a passing between the expandable part (3) and the retaining part (2,2a,2b).

15. A stent (1) according to any of the preceding claims, wherein the material is a Shape Memory Alloy metal having a transition phase above normal body cavity temperature of the human being or animal, the body cavity for which the stent (1) is intended.

16. A stent (1) according to any of the preceding claims, wherein the material is a super-elastic metal, said metal being super-elastic at normal body cavity temperature of the human being or animal, the body cavity for which the stent (1) is intended.

17. A stent (1) according to any of the preceding claims, wherein the stent (1) is made of a material being plastically deformable at normal body cavity temperature of the human being or animal, the body cavity for which the stent (1) is intended.

18. A stent (1) according to any of the preceding claims, wherein the stent (1) itself comprises a marker element being detectable from outside the body cavity by at least one of the following detections: optically, tactilely, photographically, electronically or radiologically for being able of obtaining a correct physical placement of the stent (1) in the body cavity.

19. A stent (1) according to claim 18, where the marker element is capable of marking a rotational orientation of the stent (1) in respect of a longitudinal axis of the stent (1).

20. A stent system according to claim 18 or claim 19, where the marker element is capable of marking an axial orientation of the stent (1) in respect of a longitudinal axis of the stent (1).

## Patentansprüche

1. Stent (1) zum Einführen und Anbringen im Inneren einer Körperhöhle eines Menschen oder eines Tieres, wobei der Stent (1) mit einer ersten Konfiguration während der Einführung vorgesehen ist und wobei der Stent (1) nach dem Einführen und beim Anbringen in der Körperhöhle mit einer zweiten Konfiguration vorgesehen ist, wobei der Stent (1) umfasst
- mindestens einen Halteteil (2,2a,2b) zum Aufrechterhalten eines Lumens im Inneren der Körperhöhle und
- mindestens einen ausdehnbaren Teil (3) zum Halten des Stents (1) an seinem Platz,
- wobei der mindestens eine ausdehnbare Teil eine erste kleinere Querschnittsausdehnung in der ersten Konfiguration aufweist und der mindestens eine ausdehnbare Teil eine zweite größere Querschnittsausdehnung in der zweiten Konfiguration aufweist und
- wobei der mindestens eine Halteteil in der zweiten Konfiguration eine Querschnittsausdehnung über mindestens einen Teil einer Längsausdehnung des Halteteils aufweist, die kleiner ist als die Querschnittsausdehnung mindestens eines Teils des ausdehnbaren Teils in der zweiten Konfiguration, und
- wobei der ausdehnbare Teil (3) aus mindestens einem ersten Abschnitt mit mindestens einer Spiralwindung im Uhrzeigersinn und mindestens einem zweiten Abschnitt mit mindestens einer Spiralwindung gegen den Uhrzeigersinn besteht, wobei jeder des ersten und zweiten mindestens einen Abschnitts radial ausdehnbar ist, sodass der mindestens eine erste Abschnitt während der Ausdehnung gegen den Uhrzeigersinn dreht und der mindestens eine zweite Abschnitt im Uhrzeigersinn dreht, und
- wobei sowohl der Halteteil (2,2a,2b) als auch der ausdehnbare Teil (3) zum Anbringen in einer einzelnen Körperhöhle vorgesehen sind, wobei die einzelne Körperhöhle nicht mit Körperorganen versehen ist, was zum mindestens Teilblockieren der Körperhöhle, wie Sphinktern, Membranen, Öffnungen und ähnlichen Körperorganen bestimmt ist, wobei ein Teil einer Körperhöhle ganz oder teilweise von einem anderen Teil einer Körperhöhle getrennt wird.

2. Stent (1) nach Anspruch 1, wobei mindestens ein Teil des ganzen Stents (1) durch eine Anzahl von Spiralwindungen mindestens eines Drahts ausgebildet ist.

3. Stent (1) nach Anspruch 1, wobei mindestens ein Teil des Halteteils (2,2a,2b) durch eine Anzahl von Spiralwindungen mindestens eines Drahts ausgebildet ist.

4. Stent (1) nach Anspruch 1, wobei mindestens ein Teil des ausdehnbaren Teils (3) durch eine Anzahl von Spiralwindungen mindestens eines Drahts ausgebildet ist.

5. Stent (1) nach einem der vorhergehenden Ansprüche,
- wobei der Halteteil (2,2a,2b) mindestens eine proximale Längsausdehnung und mindestens eine benachbarte distale Längsausrichtung aufweist und
- wobei sich die proximale Längsausrichtung des Halteteils (2,2a,2b) proximal zu dem ausdehnbaren Teil (3) erstreckt und
- wobei sich die benachbarte distale Längsausdehnung des Halteteils (2,2a,2b) in unmittelbarer Nähe der proximale Längsausdehnung erstreckt und
- wobei ein Querschnittsbereich der proximalen Längsausdehnung größer ist als ein Querschnittsbereich der benachbarten distalen Längsausdehnung.

6. Stent (1) nach einem der vorhergehenden Ansprüche, wobei der Stent (1) eine Verbindung (8) zwischen dem ausdehnbaren Teil (3) und dem Halteteil (2,2a,2b) aufweist und wobei die Verbindung (8) in der ersten Konfiguration des Stents (1) ein gegenseitiges Lageverhältnis des Halteteils (2,2a,2b) zu dem ausdehnbaren Teil (3) aufrechterhalten kann und die Verbindung (8) in der zweiten Konfiguration im Wesentlichen das identische gegenseitige Lageverhältnis des Halteteils (2,2a,2b) zu dem ausdehnbaren Teil (3) aufrechterhalten kann.

7. Stent (1) nach Anspruch 6, wobei die Verbindung (8) in der ersten Konfiguration des Stents (1) ein gegenseitiges Drehverhältnis des Halteteils (2,2a,2b) zu dem ausdehnbaren Teil (3) aufrechterhalten kann und die Verbindung (8) in der zweiten Konfiguration im Wesentlichen das identische gegenseitige Drehverhältnis des Halteteils (2,2a,2b) zu dem ausdehnbaren Teil (3) aufrechterhalten kann.

8. Stent (1) nach einem der vorhergehenden Ansprüche, wobei die Windungen des ausdehnbaren Teils (3) in der ersten Konfiguration eine Längsausdehnung aufweisen und wobei der ausdehnbare Teil (3) in der zweiten Konfiguration im Wesentlichen die identische Längsausdehnung aufweist wie in der ersten Konfiguration.

9. Stent (1) nach einem der vorhergehenden Ansprüche, wobei mindestens ein Teil des Halteteils durch eine Anzahl von Spiralwindungen von mindestens einem Draht ausgebildet ist und wobei der gegenseitige Abstand zwischen den Windungen weniger als 5 mm, vorzugsweise weniger als 3 mm, mehr bevorzugt 1 mm, beträgt.

10. Stent (1) nach einem der vorhergehenden Ansprüche, wobei der Werkstoff, aus dem der Stent (1) hergestellt ist, in der ersten Konfiguration eine erste Phase aufweist und in der zweiten Konfiguration eine zweite Phase aufweist und wobei eine Übergangsphase des Werkstoffs zwischen der ersten Konfiguration und der zweiten Konfiguration vorhanden ist und wobei die Einleitung des Übergangs zwischen der ersten Konfiguration und der zweiten Konfiguration durch ein Erwärmen des Stents (1) beeinflusst wird.

11. Stent (1) nach Anspruch 1-9, wobei der Werkstoff, aus dem der Stent (1) hergestellt ist, in der ersten Konfiguration eine erste Phase aufweist und in der zweiten Konfiguration eine zweite Phase aufweist und wobei eine Übergangsphase des Werkstoffs zwischen der ersten Konfiguration und der zweiten Konfiguration vorhanden ist und wobei die Einleitung des Übergangs zwischen der ersten Konfiguration und der zweiten Konfiguration durch die mechanische Freigabe des Stents (1) beeinflusst wird.

12. Stent (1) nach einem der vorhergehenden Ansprüche, wobei der Halteteil (2,2a,2b) in der Konfiguration der ersten Konfiguration eine erste Querschnittsausdehnung aufweist und wobei die erste Querschnittsausdehnung im Wesentlichen identisch mit einer zweiten Querschnittsausdehnung des Halteteils (2,2a,2b) in der Konfiguration der zweiten Konfiguration ist.

13. Stent (1) nach einem der vorhergehenden Ansprüche, wobei sich eine Mittelachse des ausdehnbaren Teils (3) und eine Mittelachse des Halteteils (2,2a,2b) in der zweiten Konfiguration des Stents (1) an einem Übergang zwischen dem ausdehnbaren Teil (3) dem Halteteil (2,2a,2b) schneiden.

14. Stent (1) nach einem der vorhergehenden Ansprüche, wobei eine Mittelachse des ausdehnbaren Teils (3) und eine Mittelachse des Halteteils (2,2a,2b) in der zweiten Konfiguration des Stents (1) an einem Übergang zwischen dem ausdehnbaren Teil (3) dem Halteteil (2,2a,2b) versetzt sind und sich nicht schneiden.

15. Stent (1) nach einem der vorhergehenden Ansprüche, wobei der Werkstoff eine Formgedächtnislegierung mit einer Übergangsphase oberhalb der normalen menschlichen oder tierischen Körperhöhlentemperatur der Körperhöhle ist, für die der Stent (1) bestimmt ist.

16. Stent (1) nach einem der vorhergehenden Ansprüche, wobei der Werkstoff ein superelastisches Metall ist, wobei das Metall bei der normalen menschlichen oder tierischen Körperhöhlentemperatur der Körperhöhle superelastisch ist, für die der Stent (1) bestimmt ist.

17. Stent (1) nach einem der vorhergehenden Ansprüche, wobei der Stent (1) aus einem Werkstoff hergestellt ist, der bei der normalen menschlichen oder tierischen Körperhöhlentemperatur der Körperhöhle plastisch verformbar ist, für die der Stent (1) bestimmt ist.

18. Stent (1) nach einem der vorhergehenden Ansprüche, wobei der Stent (1) selbst ein Markerelement umfasst, das außerhalb der Körperhöhle von mindestens einer der folgenden Erfassungsmöglichkeiten erfassbar ist: optisch, taktil, fotografisch, elektronisch oder radiologisch, um so ein korrektes physisches Anbringen des Stent (1) in der Körperhöhle zu erhalten.

19. Stent (1) nach Anspruch 18, wobei das Markerelement eine Rotationsausrichtung des Stents (1) in Bezug auf eine Längsachse des Stents (1) markieren kann.

20. Stent (1) nach Anspruch 18 oder 19, wobei das Markerelement eine axiale Ausrichtung des Stents (1) in Bezug auf eine Längsachse des Stents (1) markieren kann.

## Revendications

1. Stent (1) destiné à être inséré et mis en place à l'intérieur d'une cavité corporelle d'un être humain ou d'un animal, ledit stent (1) étant conçu pour présenter une première configuration pendant l'insertion, et ledit stent (1), après avoir été inséré et durant sa mise en place dans la cavité corporelle, étant conçu pour présenter une deuxième configuration, dans lequel ledit stent (1) comprend
- au moins une pièce de retenue (2, 2a, 2b) pour retenir une lumière à l'intérieur de la cavité corporelle, et
- au moins une pièce expansible (3) pour maintenir le stent (1) à son emplacement,
- ladite au moins une pièce expansible présentant une première extension transversale rétrécie dans la première configuration, et ladite au moins une pièce expansible présentant une deuxième extension transversale élargie dans la deuxième configuration, et
- ladite au moins une pièce de retenue, dans la deuxième configuration, présentant une extension transversale, le long d'au moins une partie d'une extension longitudinale de la pièce de retenue, qui est plus petite que l'extension transversale d'au moins une partie de la pièce expansible dans la deuxième configuration, et
- où la pièce expansible (3) est constituée d'au moins un premier tronçon d'au moins un enroulement hélicoïdal dans le sens horaire et d'au moins un deuxième tronçon d'au moins un enroulement hélicoïdal dans le sens anti-horaire, chacun desdits au moins un premier et deuxième tronçon étant radialement expansibles de manière à ce que pendant l'expansion, l'au moins un premier tronçon tourne dans le sens anti-horaire et l'au moins un deuxième tronçon tourne dans le sens horaire, et
- où la pièce de retenue (2, 2a, 2b) et la pièce expansible (3) sont toutes deux conçues pour être mises en place dans une cavité corporelle unique, ladite cavité corporelle unique ne contenant pas d'organes corporels, qui est conçu pour obstruer au moins en partie la cavité corporelle, telle que des sphincters, des membranes, des orifices et des organes corporels similaires, en obstruant en partie ou en totalité une partie d'une cavité corporelle depuis une autre partie d'une cavité corporelle.

2. Stent (1) selon la revendication 1, dans lequel au moins une partie de l'ensemble du stent (1) est constituée de plusieurs enroulements hélicoïdaux d'au moins un fil métallique.

3. Stent (1) selon la revendication 1, dans lequel au moins une partie de la pièce de retenue (2, 2a, 2b) est constituée de plusieurs enroulements hélicoïdaux d'au moins un fil métallique.

4. Stent (1) selon la revendication 1, dans lequel au moins une partie de la pièce expansible (3) est constituée de plusieurs enroulements hélicoïdaux d'au moins un fil métallique.

5. Stent (1) selon l'une quelconque des revendications précédentes,
- où la pièce de retenue (2, 2a, 2b) présente au moins une extension longitudinale proximale et présente au moins une extension longitudinale distale voisine, et
- où l'extension longitudinale proximale de la pièce de retenue (2, 2a, 2b) s'étend à proximité de la pièce expansible (3), et
- où l'extension longitudinale distale voisine de la pièce de retenue (2, 2a, 2b) s'étend dans le voisinage immédiat de l'extension longitudinale proximale, et
- où une section transversale de l'extension longitudinale proximale est plus importante qu'une section transversale de l'extension longitudinale distale voisine.

6. Stent (1) selon l'une quelconque des revendications précédentes, dans lequel le stent (1) présente une jonction (8) entre la pièce expansible (3) et la pièce de retenue (2, 2a, 2b), et dans lequel ladite jonction (8), dans la première configuration du stent (1), est capable de maintenir une relation de position mutuelle entre la pièce de retenue (2, 2a, 2b) et la pièce expansible (3), et dans lequel ladite jonction (8), dans la deuxième configuration, est capable de maintenir essentiellement la même relation de position mutuelle entre la pièce de retenue (2, 2a, 2b) et la pièce expansible (3).

7. Stent (1) selon la revendication 6, dans lequel la jonction (8), dans la première configuration, est capable de maintenir une relation de rotation mutuelle entre la pièce de retenue (2, 2a, 2b) et la pièce expansible (3), et dans lequel ladite jonction (8), dans la deuxième configuration, est capable de maintenir essentiellement la même relation de rotation mutuelle entre la pièce de retenue (2, 2a, 2b) et la pièce expansible (3).

8. Stent (1) selon l'une quelconque des revendications précédentes, dans lequel des enroulements de la pièce expansible (3), dans la première configuration, présentent une extension longitudinale, et dans lequel la pièce expansible (3), dans la deuxième configuration, présente essentiellement la même extension longitudinale que dans la première configuration.

9. Stent (1) selon l'une quelconque des revendications précédentes, dans lequel au moins une partie de la pièce de retenue est constituée de plusieurs enroulements hélicoïdaux d'au moins un fil métallique et dans lequel la distance mutuelle entre les enroulements est inférieure à 5 mm, préférentiellement inférieure à 3 mm, plus préférentiellement 1 mm.

10. Stent (1) selon l'une quelconque des revendications précédentes, dans lequel le matériau dont est constitué le stent (1) présente une première phase dans la première configuration et présente une deuxième phase dans la deuxième configuration, et où une phase de transition du matériau est présente entre la première configuration et la deuxième configuration, et où la transition entre la première configuration et la deuxième configuration peut être opérée en chauffant le stent (1).

11. Stent (1) selon les revendications 1 à 9, dans lequel le matériau dont est constitué le stent (1) présente une première phase dans la première configuration et présente une deuxième phase dans la deuxième configuration, et où une phase de transition du matériau est présente entre la première configuration et la deuxième configuration, et où la transition entre la première configuration et la deuxième configuration peut être opérée par relâchement mécanique du stent (1).

12. Stent (1) selon l'une quelconque des revendications précédentes, dans lequel la pièce de retenue (2, 2a, 2b) présente une première extension transversale, quand il est configuré dans la première configuration, et où ladite première extension transversale est essentiellement identique à une deuxième extension transversale de la pièce de retenue (2, 2a, 2b), quand il est configuré dans la deuxième configuration.

13. Stent (1) selon l'une quelconque des revendications précédentes, dans lequel un axe central de la pièce expansible (3) et un axe central de la pièce de retenue (2, 2a, 2b), quand le stent (1) est dans la deuxième configuration, se croisent au niveau d'une transition entre la pièce expansible (3) et la pièce de retenue (2, 2a, 2b).

14. Stent (1) selon l'une quelconque des revendications précédentes, dans lequel un axe central de la pièce expansible (3) et un axe central de la pièce de retenue (2, 2a, 2b), quand le stent (1) est dans la deuxième configuration, sont décalés et ne se croisent pas au niveau d'une transition entre la pièce expansible (3) et la pièce de retenue (2, 2a, 2b).

15. Stent (1) selon l'une quelconque des revendications précédentes, dans lequel le matériau est un alliage métallique à mémoire de forme présentant une phase de transition au-dessus de la température normale de la cavité corporelle de l'être humain ou de l'animal, la cavité corporelle à laquelle est destiné le stent (1).

16. Stent (1) selon l'une quelconque des revendications précédentes, dans lequel le matériau est un métal superélastique, ledit métal étant superélastique à la température normale de la cavité corporelle de l'être humain ou de l'animal, la cavité corporelle à laquelle est destiné le stent (1).

17. Stent (1) selon l'une quelconque des revendications précédentes, dans lequel le stent (1) est constitué d'un matériau plastiquement déformable à la température normale de la cavité corporelle de l'être humain ou de l'animal, la cavité corporelle à laquelle est destiné le stent (1).

18. Stent (1) selon l'une quelconque des revendications précédentes, dans lequel le stent (1) lui-même comprend un élément indicateur détectable depuis l'extérieur de la cavité corporelle par au moins l'un des modes de détection suivants : optique, tactile, photographique, électronique ou radiologique de manière à assurer une mise en place physique correcte du stent (1) dans la cavité corporelle.

19. Stent (1) selon la revendication 18, dans lequel l'élément indicateur est capable d'indiquer une orientation rotationnelle du stent (1) par rapport à un axe longitudinal du stent (1).

20. Système de stent (1) selon la revendication 18 ou la revendication 19, dans lequel l'élément indicateur est capable d'indiquer une orientation axiale du stent (1) par rapport à un axe longitudinal du stent (1).
